# EUROPEAN PATENT APPLICATION

(11) **EP 1 136 566 A1**
(43) Date of publication of application: **26.09.2001**
(21) Application number: 00870055.1
(22) Date of filing: 24.03.2000
(51) Int. Cl.: C12Q 1/68

(54) **Method and kit for detection and/or quantification of homologus nucleotide sequences on arrays**

(71) Applicant: Facultes Universitaires Notre-Dame de la Paix, 5000 Namur (BE)
(72) Inventor: Remacle, José, 5020 Malonne (BE); Hamels, Sandrine, 6280 Loverval (BE); Zammatteo, Nathalie, 5100 Jambes (BE); Lockman, Laurence, 6600 Bastogne (BE); Dufour, Sohie, 7000 Mons (BE); Alexandre, Isabelle, 5170 Lesve (BE); De Longueville, Francoise, 5100 Jambes (BE)
(74) Representative: Van Malderen, Joelle

(57) **Abstract**

The present invention is in the field of diagnosis and is related to a method and kit comprising reagents for detection and/or quantification of nucleotide sequences potentially present in a sample, said sequences being homologous after amplification by a single primer pair. The invention is especially suited for the identification and/or quantification of micro-organisms of the same genus or family or for the detection and/or quantification of related genes in a specific organism present in a biological sample.

### Annex to the application documents - subsequently filed sequences listing

SEQUENCE LISTING

<110> FACULTES UNIVERSIATIRES NOTRE-DAME DE LA PAIX

<120> METHOD AND KIT FOR DETECTION AND/OR QUANTIFICATION OF
      HOMOLOGOUS NUCLEOTIDE SEQUENCES ON ARRAYS

<130> P.FNDP.08/EP

<140> 00870055.1
<141> 2000-03-24

<160> 39

<170> PatentIn Ver. 2.1

<210> 1
<211> 23
<212> DNA
<213> Artificial Sequence

<220>
<223> Description of Artificial Sequence:
      Staphylococcus aureus 1 FemA gene primer

<400> 1
cttttgctga tcgtgatgac aaa                                          23


<210> 2
<211> 25
<212> DNA
<213> Artificial Sequence

<220>
<223> Description of Artificial Sequence:
      Staphylococcus aureus 2 FemA gene primer

<400> 2
tttatttaaa atatcacgct cttcg                                        25


<210> 3
<211> 23
<212> DNA
<213> Artificial Sequence

<220>
<223> Description of Artificial Sequence:
      Staphylococcus epidermis 1 FemA gene primer

<400> 3
tcgcggtcca gtaatagatt ata                                          23


<210> 4
<211> 22
<212> DNA
<213> Artificial Sequence

<220>

<223> Description of Artificial Sequence:
      Staphylococcus epidermis 2 FemA gene primer

<400> 4
tgcatttcca gttatttctc cc                                              22


<210> 5
<211> 24
<212> DNA
<213> Artificial Sequence

<220>
<223> Description of Artificial Sequence:
      Staphylococcus haemolyticus 1 FemA gene primer

<400> 5
attgatcatg gtattgatag atac                                            24


<210> 6
<211> 25
<212> DNA
<213> Artificial Sequence

<220>
<223> Description of Artificial Sequence:
      Staphylococcus haemolyticus 2 FemA gene primer

<400> 6
tttaatcttt ttgagtgtct tatac                                           25


<210> 7
<211> 25
<212> DNA
<213> Artificial Sequence

<220>
<223> Description of Artificial Sequence:
      Staphylococcus saprofyticus 1 FemA gene primer

<400> 7
taaaatgaaa caactcggtt ataag                                           25


<210> 8
<211> 24
<212> DNA
<213> Artificial Sequence

<220>
<223> Description of Artificial Sequence:
      Staphylococcus saprofyticus 2 FemA gene primer

<400> 8
aaactatcca taccattaag tacg                                            24


<210> 9
<211> 24

<212> DNA
<213> Artificial Sequence

<220>
<223> Description of Artificial Sequence:
      Staphylococcus hominis 1 FemA gene primer

<400> 9
cgaccagata acaaaaaagc acaa                                          24


<210> 10
<211> 22
<212> DNA
<213> Artificial Sequence

<220>
<223> Description of Artificial Sequence:
      Staphylococcus hominis 2 FemA gene primer

<400> 10
gtaattcgtt accatgttct aa                                            22


<210> 11
<211> 27
<212> DNA
<213> Artificial Sequence

<220>
<223> Description of Artificial Sequence: ATaur02 Capture
      nucleotide sequence

<400> 11
atttaaaata tcacgctctt cgtttag                                       27


<210> 12
<211> 27
<212> DNA
<213> Artificial Sequence

<220>
<223> Description of Artificial Sequence: ATepi02 Capture
      nucleotide sequence

<400> 12
attaagcaca tttctttcat tatttag                                       27


<210> 13
<211> 27
<212> DNA
<213> Artificial Sequence

<220>
<223> Description of Artificial Sequence: AThae02 Capture
      nucleotide sequence

<400> 13
atttaaagtt tcacgttcat tttgtaa                                       27

<210> 14
<211> 27
<212> DNA
<213> Artificial Sequence

<220>
<223> Description of Artificial Sequence: AThom02 Capture
      nucleotide sequence

<400> 14
atttaatgtc tgacgttctg catgaag                                          27


<210> 15
<211> 27
<212> DNA
<213> Artificial Sequence

<220>
<223> Description of Artificial Sequence: ATsap02 Capture
      nucleotide sequence

<400> 15
acttaatact tcgcgttcag cctttaa                                          27


<210> 16
<211> 24
<212> DNA
<213> Artificial Sequence

<220>
<223> Description of Artificial Sequence: FemA consensus
      primer APstap03

<400> 16
cccactcgct tatatagaat ttga                                             24


<210> 17
<211> 23
<212> DNA
<213> Artificial Sequence

<220>
<223> Description of Artificial Sequence: FemA consensus
      primer APstap04

<400> 17
ccactagcgt acatcaattt tga                                              23


<210> 18
<211> 25
<212> DNA
<213> Artificial Sequence

<220>
<223> Description of Artificial Sequence: FemA consensus

primer APstap05

<400> 18
ggtttaataa agtcaccaac atatt                                            25


<210> 19
<211> 27
<212> DNA
<213> Artificial Sequence

<220>
<223> Description of Artificial Sequence: : Ataur02 Capture
      nucleotide sequence

<400> 19
atttaaaata tcacgctctt cgtttag                                          27


<210> 20
<211> 27
<212> DNA
<213> Artificial Sequence

<220>
<223> Description of Artificial Sequence: : ATepi02 Capture
      nucleotide sequence

<400> 20
attaagcaca tttctttcat tatttag                                          27


<210> 21
<211> 47
<212> DNA
<213> Artificial Sequence

<220>
<223> Description of Artificial Sequence: : ATepi03 Capture
      nucleotide sequence

<400> 21
gaattcaaag ttgctgagaa attaagcaca tttctttcat tatttag                    47


<210> 22
<211> 67
<212> DNA
<213> Artificial Sequence

<220>
<223> Description of Artificial Sequence: : ATepi04 Capture
      nucleotide sequence

<400> 22
gaattcaaag ttgctgagaa tagttcaatg gaaggaagcg attaagcaca tttctttcat 60
tatttag                                                                67


<210> 23
<211> 87

<212> DNA
<213> Artificial Sequence

<220>
<223> Description of Artificial Sequence: : ATepi05 Capture
      nucleotide sequence

<400> 23
gaattcaaag ttgctgagaa tagttcaatg gaaggaagcg tcttcttaaa atctaaagaa 60
attaagcaca tttctttcat tatttag                                     87


<210> 24
<211> 67
<212> DNA
<213> Artificial Sequence

<220>
<223> Description of Artificial Sequence: Ataur27 Capture
      nucleotide sequence

<400> 24
gaattcaaag ttgctgagaa tagttcaatg gaaggaagcg atttaaaata tcacgctctt 60
cgtttag                                                           67


<210> 25
<211> 67
<212> DNA
<213> Artificial Sequence

<220>
<223> Description of Artificial Sequence: Atepi27 Capture
      nucleotide sequence

<400> 25
gaattcaaag ttgctgagaa tagttcaatg gaaggaagcg attaagcaca tttctttcat 60
tatttag                                                           67


<210> 26
<211> 67
<212> DNA
<213> Artificial Sequence

<220>
<223> Description of Artificial Sequence: Athae27 Capture
      nucleotide sequence

<400> 26
gaattcaaag ttgctgagaa tagttcaatg gaaggaagcg atttaaagtt tcacgttcat 60
tttgtaa                                                           67


<210> 27
<211> 67
<212> DNA
<213> Artificial Sequence

<220>
<223> Description of Artificial Sequence: Athom27 Capture

nucleotide sequence

<400> 27
gaattcaaag ttgctgagaa tagttcaatg gaaggaagcg atttaatgtc tgacgttctg 60
catgaag 67

<210> 28
<211> 67
<212> DNA
<213> Artificial Sequence

<220>
<223> Description of Artificial Sequence: Atsap27 Capture
nucleotide sequence

<400> 28
gaattcaaag ttgctgagaa tagttcaatg gaaggaagcg acttaatact tcgcgttcag 60
cctttaa 67

<210> 29
<211> 22
<212> DNA
<213> Artificial Sequence

<220>
<223> Description of Artificial Sequence: FemA fragment
consensus primer APcons3-1

<400> 29
taayaaartc accaacatay tc 22

<210> 30
<211> 22
<212> DNA
<213> Artificial Sequence

<220>
<223> Description of Artificial Sequence: FemA fragment
consensus primer APcons3-2

<400> 30
tymgntcatt tatggaagat ac 22

<210> 31
<211> 67
<212> DNA
<213> Artificial Sequence

<220>
<223> Description of Artificial Sequence: Ataur15 Capture
nucleotide sequence

<400> 31
gaattcaaag ttgctgagaa tagttcaatg gaaggaagcg tcttcttaaa atgctcttcg 60
tttagtt 67

<210> 32
<211> 65
<212> DNA
<213> Artificial Sequence

<220>
<223> Description of Artificial Sequence: Ataur27 Capture
      nucleotide sequence

<400> 32
gaattcaaag ttgctgagaa tagttcaatg gaaggaagcg atttaaaata tcgctcttcg 60
tttag                                                             65


<210> 33
<211> 67
<212> DNA
<213> Artificial Sequence

<220>
<223> Description of Artificial Sequence: Ataur40 Capture
      nucleotide sequence

<400> 33
gaattcaaag ttgctgagaa tagttcaaat ctttatttaa aatatcacgc tcttcgttta 60
gttcttt                                                           67


<210> 34
<211> 67
<212> DNA
<213> Artificial Sequence

<220>
<223> Description of Artificial Sequence: Atana15 Capture
      nucleotide sequence

<400> 34
gaattcaaag ttgctgagaa tagttcaatg gaaggaagcg tcttcttaaa atgctcttca 60
tttagtt                                                           67


<210> 35
<211> 67
<212> DNA
<213> Artificial Sequence

<220>
<223> Description of Artificial Sequence: Atana27 Capture
      nucleotide sequence

<400> 35
gaattcaaag ttgctgagaa tagttcaatg gaaggaagcg gtttaaaata tcacgctctt 60
catttag                                                           67


<210> 36
<211> 67
<212> DNA
<213> Artificial Sequence

<220>
<223> Description of Artificial Sequence: Atana40 Capture
      nucleotide sequence

<400> 36
gaattcaaag ttgctgagaa tagttcaaat ctttgtttaa aatatcacgc tcttcattta 60
gttcttt                                                           67


<210> 37
<211> 67
<212> DNA
<213> Artificial Sequence

<220>
<223> Description of Artificial Sequence: Atepi15 Capture
      nucleotide sequence

<400> 37
gaattcaaag ttgctgagaa tagttcaatg gaaggaagcg tcttcttaaa attttcatta 60
tttagtt                                                           67


<210> 38
<211> 67
<212> DNA
<213> Artificial Sequence

<220>
<223> Description of Artificial Sequence: Atepi27 Capture
      nucleotide sequence

<400> 38
gaattcaaag ttgctgagaa tagttcaatg gaaggaagcg attaagcaca tttctttcat 60
tatttag                                                           67


<210> 39
<211> 67
<212> DNA
<213> Artificial Sequence

<220>
<223> Description of Artificial Sequence: Atepi40 Capture
      nucleotide sequence

<400> 39
gaattcaaag ttgctgagaa tagttcaaat ctttattaag cacatttctt tcattattta 60
gttcctc                                                           67

## Description

### Field of the invention

The present invention is in the field of diagnosis and is related to a method and kit comprising reagents for detection and/or quantification of nucleotide sequences potentially present in a sample, said sequences being homologous after amplification by a single primer pair.

The invention is especially suited for the identification and/or quantification of micro-organisms of the same genus or family or for the detection and/or quantification of related genes in a specific organism present in a biological sample.

### Background of the invention

The development of the biochips technology allows the detection of multiple nucleotide sequences simultaneously in a given assay. Arrays are solid supports containing on their surface a series of discrete regions bearing capture nucleotide sequences (or probes) that are able to bind (by hybridisation) to a corresponding target nucleotide sequence(s) possibly present in a sample to be analysed. If the target sequence is labelled with modified nucleotides during a reverse transcription or an amplification of said sequence, then a signal can be detected and measured at the binding location. Its intensity gives an estimation of the amount of target sequences present in the sample. Such technology allows the identification and/or quantification of genes or species for diagnostic or screening purpose.

### State of the art

The Company Affymetrix Inc. has developed a method for direct synthesis of oligonucleotides upon a solid support, at specific locations by using masks at each step of the processing. Said method comprises the addition of a new nucleotide on a growing oligonucleotide in order to obtain a desired sequence at a desired location. This method is derived from the photolithographic technology and is coupled with the use of photoprotective groups, which are released before a new nucleotide is added (EP-A1-0476014, US-A-5,445,934, US-A-5,143,854 and US-5,510,270). However, only small oligonucleotides are present on the surface, and said method finds applications mainly for sequencing or identifying nucleotide sequences in a pattern of positive spots corresponding to each specific oligonucleotide bound on the array. The identification of target sequences is thereafter made by comparison of said pattern with a reference. Said technique was applied to the identification of Mycobacterium tuberculosis rpoB gene (WO97/29212 and WO98/28444), wherein the capture nucleotide sequence comprises less than 30 nucleotides. Said method allows an analysis of two different sequences that may differ by a single nucleotide. Small capture nucleotide sequences (having a length comprised between 10 and 20 nucleotides) are preferred since the discrimination between two oligonucleotides differing in one base is higher when their length is smaller. Said last method is suitable for the identification of Single Nucleotide Polymorphism (SNP) or for genotyping.

However, for longer target fragments (like the cDNA copy of mRNA), different fragments can recognise the same probe and the rate of hybridisation is lower. Therefore, the fragments are cut into smaller species and the method requires the use of several capture nucleotide sequences in order to obtain a pattern of signals which attest the presence of a given gene (WO97/10364 and WO97/27317). Short capture nucleotide sequences are used with few nucleotides, preferably about 45 bases, and in practice, between 10 and 25 nucleotides per probe. Said cutting also decreases the number of labelled nucleotides, and thus reduces the obtained signal.

The US patent 5,605,662 describes the binding of full nucleotide sequences on plots of electronic based chips, each of the plots bearing a specific oligonucleotide sequence. In order to allow SNP determination, a target nucleotide sequence is spotted on the chips and specific small labelled nucleotides are then incubated in order to determine their fixation on the target. A change in the plot charge allows a discrimination of the different nucleotide differing by one or more bases and a specific SNP analysis (Gilles et al. 1999, Nature Biotechnology 17, p. 365 (1999)).

The international patent application WO94/22889 describes electronic chips made of preconstructed oligonucleotides bearing a pyrol group, that after an electropolymerisation, allows the formation of a conducting polymer. This method is particularly suitable for the preparation of electronic chips bearing small capture nucleotide sequences.

However, the known approaches using either small or long capture nucleotide sequences fixed on arrays are not optimal when applied for a diagnostic purpose, especially for the detection and possibly the quantification of different sequences presenting a high homology between them and that should be detected at the same time upon the same array of a solid support.

### Aims of the invention

The present invention aims to provide a new method and device to improve the detection and possibly the quantification of nucleotide sequences, preferably multiple homologous nucleotide sequences, coming from different or the same organism, by hybridisation on single stranded capture nucleotide sequences and that does not present the drawbacks of the state of the art.

### Definitions

The terms "nucleic acid, oligonucleotide, array, probe, target nucleic acid, bind substantially, hybridising specifically to, background, quantifying" are the ones described in the international patent application WO97/27317 incorporated herein by reference.

The terms "nucleotide triphosphate, nucleotide, primer sequence" are those described in the European patent application 99870226.0 incorporated herein by reference.

"Homologous sequences" mean nucleotide sequences having a percentage of nucleotides identical at corresponding positions which is higher than in purely random alignments. They are considered as homologous when they show a minimum of homology (or sequence identity) defined as the percentage of identical nucleotides found at each position compared to the total nucleotides, after the sequences have been optimally aligned taking into account additions or deletions (like gaps) in one of the two sequences to be compared. Genes coding for a given protein but present in genetically different sources like different organisms are usually homologous. Also in a given organism, genes coding for proteins or enzymes of the same family (Interleukins, cytochrome b, p. 450). The degree of homology (or sequence identity) can vary a lot as homologous sequences may be homologous only in one part, a few parts or portions or all along their sequences. The parts or portions of the sequences that are identical in both sequences are said conserved. Protein domains which present a conserved three dimensional structure are usually coded by homologous sequences and even often by a unique exon. The sequences showing a high degree of invariance in their sequences are said to be highly conserved and they present a high degree of homology.

Methods of alignment of sequences are based on local homology algorithms which have been computerised and are available as for example (but not limited to) Clustal® , (Intelligenetics, Mountain Views, California), or GAP® , BESTFIT® , FASTA® and TFASTA® (Wisconsin Genetics Software Package, Genetics Computer Group Madison, Wisconsin, USA) or Boxshade® .

The term "consensus sequence" is a sequence determined after alignment of the several homologous sequences to be considered (calculated as the base which is the most commonly found in each position in the compared, aligned, homologous sequences).

The consensus sequence represents a sort of «average» sequence which is as close as possible from all the compared sequences. For high homologous sequences or if the consensus sequence is long enough and the reaction conditions are not too stringent, it can bind to all the homologous sequences. This is especially usefull for the amplification of homologous sequences with the same primers called, consensus primers. Experimentally, the consensus sequence calculated from the programs above can be adapted in order to obtain such property.

### Summary of the invention

The inventors have discovered that it is possible to provide both a sensitive and specific detection method, combined or not with a quantification, for (possible multiple homologous) nucleotide sequences present in a sample by their discrimination upon an array.

The inventors have found that it is possible to discriminate homologous target sequences upon an array by using bound capture nucleotide sequences composed of at least two parts, one being a spacer bound to the support and the other part being a specific nucleotide sequence able to hybridise with the nucleotide target sequence.

Furthermore, said detection is greatly increased if high concentrations of capture nucleotide sequences are bound to the surface of the solid support.

The present invention is related to a detection and/or quantification method of target nucleotide sequences, possibly homologous to each other(s), and present in a biological sample, comprising the steps of:
- possibly extracting original nucleotide sequences 1 from the biological sample;
- possibly cutting (by RNase, nuclease, restrictions enzymes or other means, such as increase of temperature,...), copying and/or amplifying (preferably all or most of the homologous sequences are amplified by using the same primer sequence(s)) 6 at least a part of the original nucleotide sequences 1 present in the biological sample into target nucleotide sequences 2;
- putting into contact the obtained and possibly previously labelled target nucleotide sequences 2 with single stranded capture nucleotide sequences 3 bound to an insoluble solid support 4, said single stranded capture nucleotide sequences 3 containing a sequence 5 between about 10 and about 60 bases specific for the target nucleotide sequences 2; said single stranded capture nucleotide sequences having a total length comprised between about 30 and about 600 bases and being bound to the insoluble support 4 surface according to an array with a density of at least 4, 10, 16, 20, 50, 100, 1000, 4000, 10 000 or more different single stranded capture nucleotide sequences/cm² of solid support surface; and
- detecting (and possibly quantifying) a signal resulting from the formation of double stranded nucleotide sequences after hybridisation between the target and the capture nucleotide sequences 2 and 3 by complementary base pairing.

According to the invention, the most common copy method for eucaryotypes is the retrotranscription of the mRNA using a poly-dT primer which recognises the poly-A sequence of all the mRNA so that they are all copied and can then be detected.

The method according to the invention further comprises the step of correlating the signal of detection to the presence of specific microorganism(s), or genetic characteristics (polymorphism, diagnostic predisposition or evolution (monitoring) of genetic diseases, including cancer, ...) of a patient (including the human) from which the biological sample has been obtained. The biological sample can be also any culture medium wherein microorganisms, xenobiotics or pollutants are present, or an extract obtained from a plant or an animal (including a human) organ, tissue, cell or biological fluid (blood, serum, urine, etc).

The method according to the invention can be performed by using a specific diagnostic and/or quantification kit or device comprising at least an insoluble solid support 4 upon which are bound single stranded capture nucleotide sequences 3 (preferably bound to the surface of the solid support 4 by a direct covalent link or by the intermediate of a spacer 7 (made of at least 4 carbons chain) according to an array with a density of at least 4, and preferably at least 10, 16, 20, 50, 100, 1000, 4000 or more than 10 000, different single stranded capture nucleotide sequences/cm² insoluble solid support 4 surface; said single stranded capture nucleotide sequences 3 having advantageously a length comprised between about 30 and about 600 bases and containing a sequence 5 of about 10 to about 60 bases and being specific for the target nucleotide sequences 2 (which means that said bases of said sequences are able to form a binding with their complementary bases upon the sequence of the target sequences 2 by hybridisation).

In the method, kit and device according to the invention, the detection sensitivity can be greatly increased if capture nucleotide sequences are spotted to the solid support surface by a robot, at high density according to an array. A preferred embodiment of the invention is to use amounts of capture nucleotide sequences 3 spotted according to an array resulting to the fixation (binding) of between 0.01 to 5 pmoles of sequence equivalent/cm² of solid support 4 surface.

The kit or device according to the invention may also incorporate various media or devices for performing the method according to the invention. Said kit (or device) can also be included in an automatic apparatus such as a high-throughput screening apparatus for the detection and/or the quantification of multiple nucleotide sequences present in a biological sample to be analysed. Said kit or apparatus can be adapted for performing all the steps or only several specific steps of the method according to the invention.

In the method, the kit (device) or apparatus according to the invention, the length of the bound capture nucleotide sequences is preferably comprised between about 30 and about 600 bases, preferably between about 40 and about 400 bases and more preferably between about 40 and about 100 bases. Longer nucleotide sequences can be used if they do not lower the binding yield of the target nucleotide sequences usually by adopting hairpin based secondary structure or by interaction with each other.

Target sequences can be maintained short if stopper primer are incorporated into the solution so as to stop the retotranscription at the chosen sequence making the transcript of desired sequence. Short sequences of DNA can also be obtained by cutting (specific cleavage with nucleases,restriction enzymes and small sequences of RNA by RNAses or by non specific cleavage by heating or in the presence of a base (WO97/10365)). A further step of cutting the probes into smaller fragments after amplification can be added. However, the signal is reduced when targets are labelled by incorporation of labelled nucleotides during their copy or amplification since much less labels are present on targets pieces once cut. Furthermore, the cutting intensity and efficiency can vary from one experiment to the other and this can create problem for the reproduction of routine assays.

If the homology between the sequences to be detected is low (between 30 and 60%), parts of the sequence which are specific in each sequence can be used for the design of specific capture nucleotide sequences binding each of the different target sequences. However, it is more difficult to find part of the sequence sufficiently conserved as to design "consensus" sequences which will amplify or copy all desired sequences. If one pair of consensus primers is not enough to amplify all the homologous sequences, then a mixture of two or more primers pairs is added in order to obtain the desired amplifications. The minimum homologous sequences amplified by the same consensus primer is two, but there is no limitation to said number.

If the sequences show high degree of homology, higher than 60% and even higher than 90%, then the finding of common sequence for consensus primer is easily obtained, but the choice for specific capture nucleotide sequences become more difficult.

In another preferred embodiment of the invention, the capture nucleotide sequences are chemically synthesised oligonucleotides sequences shorter than 100 bases (easily performed on programmed automatic synthesiser). Such sequences can bear a functionalised group for covalent attachment upon the support, at high concentrations.

Longer capture nucleotide sequences are preferably synthesised by PCR amplification (of a sequence incorporated into a plasmid containing the specific part of the capture nucleotide sequence and the non specific part (spacer)).

In a further embodiment of the invention, the specific sequence of the capture nucleotide sequence is separated from the surface of the solid support by at least about 6.8 nm long, equivalent to the distance of at least 20 base pair long nucleotides in double helix form.

In the method, kit (device) or apparatus according to the invention, the part (or parties) of the capture nucleotide sequences complementary to the target nucleotide sequence is comprised between about 10 and about 60 bases, preferably between about 15 and about 40 bases and more preferably between about 20 and about 30 bases. These bases are preferably assigned as a continuous sequence located at or near the extremity of the capture nucleotide sequence. This sequence is considered as the specific sequence for the detection. In a preferred form of the invention, the sequence located between the specific capture nucleotide sequence and the support is a non specific sequence.

In another embodiment of the invention, a specific sequence comprising between about 10 and about 60 bases, preferably between about 15 and about 40 bases and more preferably between about 20 and about 30 bases is located on a capture nucleotide sequence comprising a sequence between about 30 and about 600 bases.

Several portion sequences specific of the target sequence, present on the capture nucleotide sequences and having the capacity to bind a target sequence, may be present on the array. These specific portion sequences can be identically repeated on the same capture nucleotide sequence. Preferably, two or more specific portion sequences are present on the same one or different capture nucleotide sequences. Preferentially, these different specific capture nucleotide sequences are present in the same spot of the array or in different spots.

The method, kit (device) or apparatus according to the invention are suitable for the detection and/or the quantification of target nucleotide sequences which are made of DNA or RNA, including sequences which are partially or totally homologous upon their total length.

The method according to the invention can be performed even when the different target nucleotide sequences present between them an homology (or sequence identity) greater than 30%, greater than 60% and even greater than 80%.

In the method, kit (device) or apparatus according to the invention, the capture nucleotide sequences are advantageously covalently bound (or fixed) upon the insoluble solid support, preferably by one of their extremities as described hereafter.

The method according to the invention gives significant results which allows detection and quantification with amplicons in solutions at concentration of lower than about 10 nM, of lower than about 1 nM, preferably of lower than about 0.1 nM and more preferably of lower than about 0.01 nM (= 1 fmole/100 *µ*l).

Another important aspect of this invention is to use very concentrate capture nucleotide sequences on the surface. If too low, the yield of the binding is quickly lower and is undetectable. Concentrations of capture nucleotide sequences between 600 and 3,000 nM in the spotting solutions are preferred. However, concentrations as low as 100 nM still give positive results in favourable cases (when the yield of covalent fixation is high or when the target to be detected is single stranded and present in high concentrations). Such low spotting concentrations would give density of capture nucleotide sequence as low as 3 fmoles per cm². However, the preferred embodiment of this invention is to use concentration of at least 10 fmoles per cm² and more preferably of at least 100 fmoles per cm². On the other side, higher density was only limited in the assays by the concentrations of the capture solutions, but concentrations still higher than 3,000 nM give good results.

The use of these very high concentrations and long probes are two unexpected characteristic features of the invention. The theory of DNA hybridisation proposed that the rate of hybridisation between two DNA complementary sequences in solution is proportional to the square root of the DNA length, the smaller one being the limited factor (Wetmur, J.G. and Davidson, N. 1968, J. Mol. Biol. 3, 584). In order to obtain the required specificity, the specific sequences of the capture nucleotide sequences had to be small compared to the target. Moreover, the targets were obtained after PCR amplification and were double stranded so that they reassociate in solution much faster than to hybridise on small sequences fixed on a solid support where diffusion is low thus reducing even more the rate of reaction. It was unexpected to observe a so large increase in the yield of hybridisation with the same short specific sequence.

The amount of target sequences which "bind" on the spots are very small compared to the amount of capture nucleotide sequences present. So there is a large excess of capture nucleotide sequence and there was no reason to obtain the binding if even more capture nucleotide sequences.

One may perform the detection on the full length sequence after amplification or copy and when labelling is performed by incorporation of labelled nucleotides, more markers are present on the hybridised target making the assay sensitive. The labelled target sequences can also be first cut into smaller pieces before hybridisation.

The method, kit and apparatus according to the invention may comprise the use of other bound capture nucleotide sequences, which may have the same characteristics as the previous ones and may be used to detect another group of homologous sequences (preferably amplified by common primer(s)).

In the microbiological field, one may use consensus primer(s) specific for each family, or genus, of microorganisms and then identify some or all the species of these various families in an array by using capture nucleotide sequences of the invention. Detection of other non homologous sequences can be advantageously performed on the same array (i.e. by allowing an hybridisation with a standard nucleotide sequence used for the quantification, with consensus capture nucleotide sequences for the same or different micro-organisms strains, with a sequence allowing a detection of a possible antibiotic resistance gene by micro-organisms, for positive or negative control of hybridisation or just for other detection of other sequences present in the sample). Said other capture nucleotide sequences have (possibly) a specific sequence comprised between 10 and 60 bases or longer and a total length as high as 600 bases and are also bound upon the insoluble solid support (preferably in the array made with the other bound capture nucleotide sequences related to the invention). A long capture nucleotide sequence may also be present on the array as consensus capture nucleotide sequence for hybridisation with all sequences of the microorganisms from the same family or genus, thus giving the information on the presence or not of a microorganism of such family, genus in the biological sample.

The same array can also bear capture nucleotide sequences specific for a bacterial group (Gram positive or Gram negative strains or even all the bacteria).

Another application is the detection of homologous genes from a consensus protein of the same species, such as various isoforms of cytochromes P450 by specific capture nucleotide sequences with or without the presence of a consensus capture nucleotide sequence for all the cytochromes possibly present in a biological sample. Such isoform detection is performed at the gene level by retrotranscription into cDNA.

The solid support according to the invention can be or can be made with materials selected from the group consisting of gel layers, filters (Nylon, nitrocellulose, etc), glasses, electronic devices, silicon or plastic support, polymers, compact discs, metallic supports or a mixture thereof (see EP 0 535 242, US 5,736,257, WO99/35499, US 5,552,270, etc). Advantageously, said solid support is a single glass slide which may comprise additional means (barcodes, markers, etc.) or media for improving the method according to the invention.

The amplification step used in the method according to the invention is advantageously obtained by well known amplification protocols, preferably selected from the group consisting of PCR, RT-PCR, LCR, CPT, NASBA, ICR or Avalanche DNA techniques.

Advantageously, the target nucleotide sequences to be detected and/or quantified are labelled previously to their hybridisation with the single stranded capture nucleotide sequences. Said labelling (with known techniques from the person skilled in the art) is preferably also obtained upon the amplified sequence previously to the denaturation (if the method includes an amplification step).

Advantageously, the length of the target nucleotide sequences is selected as being of a limited length preferably between 100 and 200 bases, preferably between 100 and 400 bases and more preferably between 100 and 800 bases. This preferred requirement depends on the possibility to find consensus primers to amplify the required sequences possibly present in the sample. Too long target may reallocate faster and adopt secondary structures which can inhibit the fixation on the capture nucleotide sequences.

The length of said target nucleotide sequences to be detected and/or quantified can be also determined by the use of specific primer and stopper sequences for the retro-transcription of the original biological nucleotide sequences (especially for RNA sequences). Advantageously, said RNA sequences are 16S and 23S rRNA sequences or 18S and 25S rRNA sequences.

The assay for RNA present in large amount like the 16s or 23s rRNA in procaryote cells or the 18s or 25s rRNA in eucaryote cells does not usually require an amplification step (RNA sequences are present in multiple copies in cells). The extremity of the RNA is copied by retro-transcription by using eiher one primer specific of each sequence or common to some or all of them, that will hybridise at a short distance from the extremity. However it is also possible to copy one specific part of the RNA by using a primer to start the copy and a blocking oligonucleotide which binds to the RNA and stops the copying by the reverse transcriptase. This blocking oligonucleotide has its 3' end blocked so that it can not be used by the transcriptase to start a copy. The most simple 3' block being a deoxy 3' carbon (but other one like the presence of a pyrophosphate at the 3' carbon is also working). The 5' end of this blocking oligonucleotides is also NH2 labelled.

Detection of mRNA is also a preferred application of this invention given the fact that there are hundreds or thousand different mRNA in any living cells, which, in eukariotic cells, possess a poly-A tail which can serve for simultaneous copy of these sequences.

The detection of polymorphism sequences (which can be considered as homologous even if differing by only one base) can be made also by the method according to the invention.

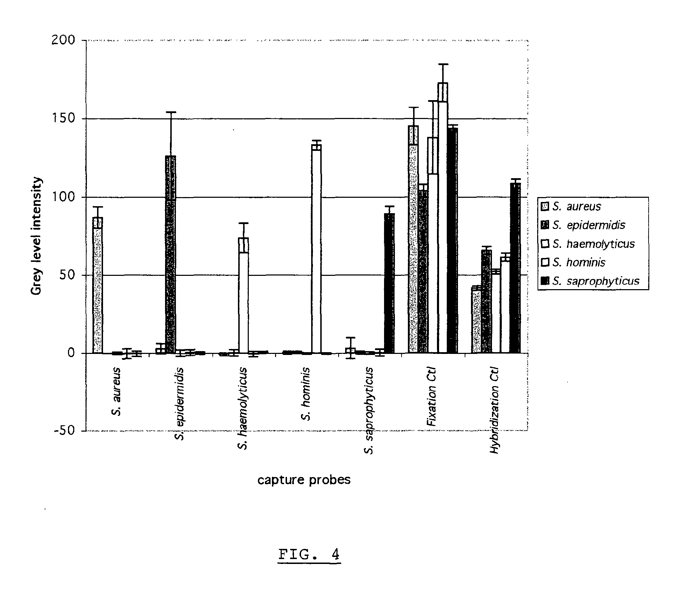

According to a further aspect of the present invention, the method, kit (device) or apparatus according to the invention is advantageously used for the detection and/or the quantification of different Staphylococcus species or variants, preferably the *S*. *aureus, the S. epidermidis, the S. saprophyticus, the S*. *hominis or the S*. *haemolyticus* present together or separately in a biological sample, said detection being obtained by detecting the genetic variants of the FemA gene in said different species, preferably by using a common locations in the *FemA* genetic sequence. Advantageously the identification of these Staphylococcus species is performed simultaneously on an array in the presence of a long capture nucleotide sequence which bind the *FemA* sequences from all *Staphylococcus* species. This capture nucleotide sequence has been designed in order to bind in the conditions used for the hybridisation to the *FemA* amplified by the consensus primers used for the specific identification. In this way two informations are obtained with the same PCR products : is there any *Staphylococcus* present in the sample and secondly is it one of the five species specific capture nucleotide sequence present on the array. In the figure 4, the capture nucleotide sequence could detect all of the 15 *Staphylococcus* species tested (and probably from all of the *Staphylococcus* species). It can be then considered as a capture nucleotide sequence for the Staphylococcus genus or family. Advantageously, the array also bears a capture nucleotide sequence for the detection of the *MecA* gene (resistance to β-lactame antibiotics), a positive control for testing the efficiency of hybridisation, a negative control and other control means for testing the binding of the capture nucleotide sequences on the support.

Preferably, the primer(s) and the specific portions of said *FemA* sequence used for obtaining amplified products are the ones described hereafter in example 2. These primers have been chosen as consensus primers for the amplification of the *FemA* genes of all of the 16 *Staphylococcus* tested and they probably will amplified the *FemA* from all the *Staphylococcus* species.

After hybridisation on the array, the target sequences can be detected by current techniques. Without labelling, preferred methods are the identification of the target by mass spectrometry now adapted to the arrays (US-A-5,821,060) or by intercalating agents followed by fluorescent detection(WO97/27329 or Fodor et al., Nature 364, p. 555 (1993)).

The labelled associated detections are numerous. A review of the different labelling molecules is given in W0 97/27317. They are obtained using either already labelled primer or by incorporation of labelled nucleotides during the copy or amplification step. A labelling can also be obtained by ligating a detectable moiety onto the RNA or DNA to be tested (a labelled oligonucleotide, which is ligated, at the end of the sequence by a ligase). Fragments of RNA or DNA can also incorporate labelled nucleotides at their 5'OH or 3'OH ends using a kinase, a transferase or a similar enzyme.

The most frequently used labels are fluorochromes like Cy3, Cy5 and Cy7 suitable for analysing an array by using commercially available array scanners (General Scanning, Genetic Microsystem, ...). Radioactive labelling, cold labelling or indirect labelling with small molecules recognised thereafter by specific ligands (streptavidin or antibodies) are common methods. The resulting signal of target fixation on the array is either fluorescent, colorimetric, diffusion, electroluminescent, bio- or chemiluminescent, magnetic, electric like impedometric or voltametric (US-A-5,312,527). A preferred method is based upon the use of the gold labelling of the bound target in order to obtain a precipitate or silver staining which is then easily detected and quantified by a colorimetric detector.

Quantification has to take into account not only the hybridisation yield and detection scale on the array (which is identical for target and reference sequences) but also the extraction, the amplification (or copying) and the labelling steps.

The method according to the invention may also comprise means for obtaining a quantification of target nucleotide sequences by using a standard nucleotide sequence (external or internal standard) added at known concentration. A capture nucleotide sequence is also present on the array so as to fix the standard in the same conditions as said target nucleotide sequences (possibly after amplification or copying); the method comprising the step of quantification of a signal resulting from the formation of a double stranded nucleotide sequence formed by complementary base pairing between the capture nucleotide sequences and the standard nucleotide sequences and the step of a correlation analysis of signal resulting from the formation of said double stranded nucleotide sequence and the signal resulting from the double stranded nucleotide sequence formed by complementary base pairing between capture nucleotide sequence(s) and a target nucleotide sequence in order to quantify the presence of the original nucleotide sequence to be detected and/or quantified in the biological sample.

Advantageously the standard is added in the initial biological sample or after the extraction step and is amplified or copied with the same primers and/or has a length and a GC content identical or differing from no more than 20% to the target. More preferably, the standard can be designed as a competitive internal standard having the characteristics of the internal standard found in the document WO98/11253. Said internal standard has a part of its sequence common to the target sequence and a specific part which is different. It also has at or near its two ends sequences which are complementary of the two primers used for amplification or copy of the target and similar GC content (WO98/11253). In the preferred embodiment of this invention, the common part of the standard and the target, means a nucleotide sequence which is homologous to all the target nucleotide sequences amplified by the same primers (i.e. which belong to the same family or organisms to be quantified).

Preferably, the hybridisation yield of the standard through this specific sequence is identical or differ no more than 20% from the hybridisation yield of the target sequence and quantification is obtained as described in WO 98/11253.

Said standard nucleotide sequence, external and/or internal standard, is also advantageously included in the kit (device) or apparatus according to the invention, possibly with all the media and means necessary for performing the different steps according to the invention (hybridisation and culture media, polymerase and other enzymes, standard sequence(s), labelling molecule(s), etc.).

Advantageously, the biochips also contain spots with various concentrations (i.e. 4) of labelled capture nucleotide sequences. These labelled capture nucleotide sequences are spotted from known concentrations solutions and their signals allow the conversion of the results of hybridisation into absolute amounts. They also allow to test for the reproducibility of the detection.

The solid support (biochip) can be inserted in a support connected to another chamber and automatic machine through the control of liquid solution based upon the use of microfluidic technology. By being inserted into such a microlaboratory system, it can be incubated, heated, washed and labelled by automates, even for previous steps (like extraction of DNA, amplification by PCR) or the following step (labelling and detection). All these steps can be performed upon the same solid support.

The present invention will be described in details in the following non-limiting examples in reference to the enclosed figures.

### Brief description of the drawings

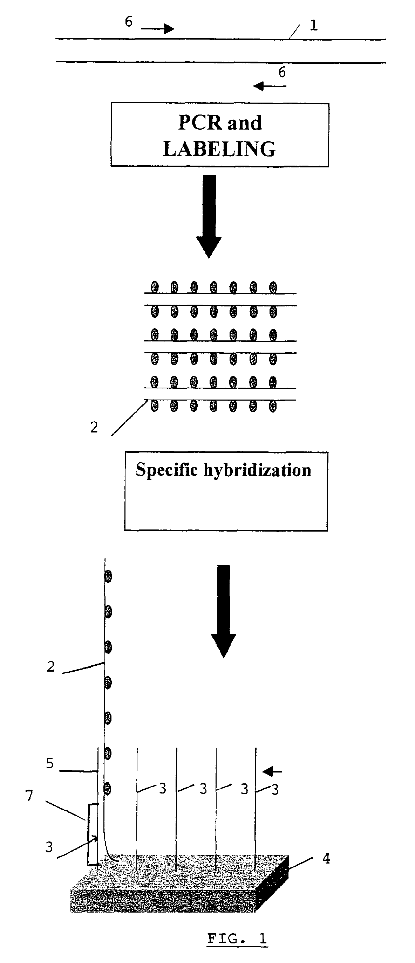

Figure 1 is a schematic presentation of the detection and identification of *5 Staphylococcus* species on a biochips according to the invention after PCR amplification with consensus primers.

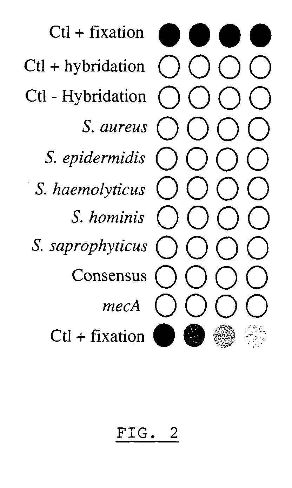

Figure 2 represents the design of an array which allows the determination of the 5 most common *Staphylococcus* species, of the presence of any Staphylococcus strain and of the MecA gene.

Figure 3 shows the modification of various parameters improving the method according to the invention.

Figure 4 shows the summary of detection of various *Staphylococcus* species.

Figure 5 presents the effect of the length of the specific sequence of the capture nucleotide sequence on the discrimination between sequences with different level of homology.

Figure 6 shows the sensitivity obtained for the detection of *FemA* sequences from S. *aureus* on array bearing the small specific capture nucleotide sequence for a *S*. *aureus* and a consensus sequence.

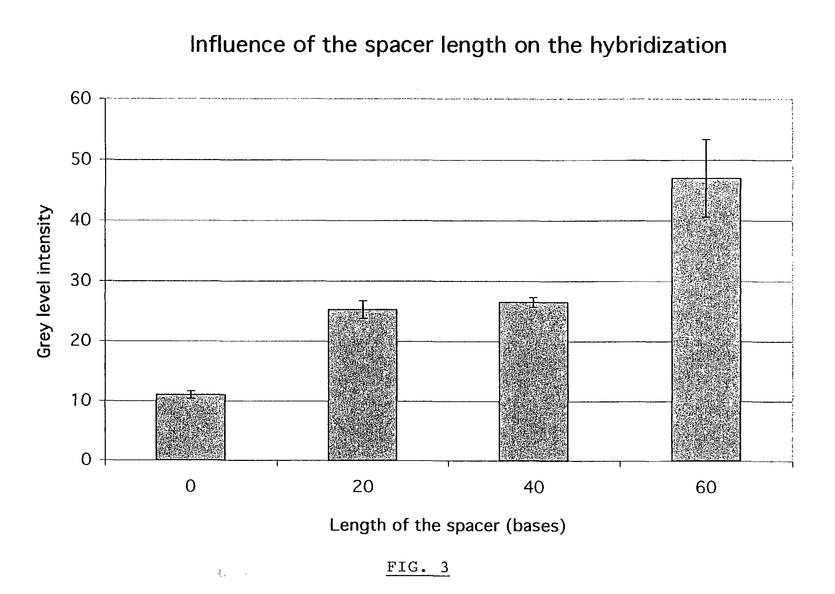

### Example 1: Detection of homologous FemA sequences on array bearing long specific capture nucleotide sequences (Fig. 3)

### Production of the capture nucleotide sequences and of the targets

The *FemA* genes corresponding to the different *Staphylococci* species were amplified separately by PCR using the following primers:

S. aureus 1 : 5′ CTTTTGCTGATCGTGATGACAAA 3′
S. aureus 2 : 5′ TTTATTTAAAATATCACGCTCTTCG 3′
S. epidermidis 1 : 5′ TCGCGGTCCAGTAATAGATTATA 3′
S. epidermidis 2 : 5′ TGCATTTCCAGTTATTTCTCCC 3′
S. haemolyticus 1 : 5′ ATTGATCATGGTATTGATAGATAC 3′
S. haemolyticus 2 : 5′ TTTAATCTTTTTGAGTGTCTTATAC 3′
S. saprophyticus 1 : 5′ TAAAATGAAACAACTCGGTTATAAG 3′
S. saprophyticus 2 : 5′ AAACTATCCATACCATTAAGTACG 3′
S. hominis 1 : 5′ CGACCAGATAACAAAAAGCACAA 3′
S. hominis 2 : 5′ GTAATTCGTTACCATGTTCTAA 3′

The PCR was performed in a final volume of 50 *µ*l containing: 1.5 mM MgCl₂, 10 mM Tris pH 8.4, 50 mM KCl, 0.8 µM of each primer, 50 µM of each dNTP, 50 *µ*M of biotin-16-dUTP), 1.5 U of Taq DNA polymerase Biotools, 7.5% DMSO, 5 ng of plasmid containing FemA gene. Samples were first denatured at 94 °C for 3 min. Then 40 cycles of amplification were performed consisting of 30 sec at 94 °C, 30 sec at 60 °C and 30 sec at 72 °C and a final extension step of 10 min at 72 °C. Water controls were used as negative controls of the amplification. The sizes of the amplicons obtained using these primers were 108 bp for *S*. *saprophyticus,* 139 bp for *S*. *aureus,* 118 bp for *S*. *hominis,* 101 pb for *S. epidermidis* and 128 bp for *S*. *haemolyticus.* The sequences of the capture nucleotide sequences were the same as the corresponding amplicons but they were single strands.

The biochips also contains positive controls which were CMV amplicons hybridised on their corresponding capture nucleotide sequence and negative controls which were capture nucleotide sequences for a HIV-I sequence on which the CMV could not bind.

### Capture nucleotide sequence immobilisation

The protocol described by Schena et al (Proc. Natl Acad. Sci. USA 93, 10614 (1996)) was followed for the grafting of aminated DNA to aldehyde derivatised glass. The aminated capture nucleotide sequences were spotted from solutions at concentrations ranging from 150 to 3000 nM. The capture nucleotide sequences were printed onto the silylated microscopic slides with a home made robotic device (250 *µ*m pins from Genetix (UK) and silylated (aldehyde) microscope slides from Cell associates (Houston, USA)). The spots have 400 *µ*m in diameter and the volume dispensed is about 0,5 nl. Slides were dried at room temperature and stored at 4 °C until used.

### Hybridisation

At 65 *µ*l of hybridisation solution (AAT, Namur, Belgium) were added 5 *µ*l of amplicons and the solution was loaded on the array framed by an hybridisation chamber. For positive controls we added 2 nM biotinylated CMV amplicons of 437 bp to the solution; their corresponding capture nucleotide sequences were spotted on the array. The chamber was closed with a covership and slides were denatured at 95 °C for 5 min. The hybridisation was carried out at 60° for 2 h. Samples were washed 4 times with a washing buffer.

### Colorimetric detection

The glass samples were incubated 45 min at room temperature with 800 *µ*l of streptavidin labelled with colloidal gold 1000 x diluted in blocking buffer (Maleic buffer 100 mM pH 7.5, NaCl 150 mM, Gloria milk powder 0.1%). After 5 washes with washing buffer, the presence of gold served for catalysis of silver reduction using a staining revelation solution (AAT, Namur, Belgium). The slides were incubated 3 times 10 min with 800 *µ*l of revelation mixture, then rinsed with water, dried and analysed using a microarray reader. Each slides were then quantified by a specific quantification software.

### Fluorescence detection

The glass samples were incubated 45 min at room temperature with 800 *µ*l of Cyanin 3 or Cyanin 5 labelled streptavidin. After washing the slides were dried before being stored at room temperature. The detection was performed in the array-scanner GSM 418 (Genetic Microsystem, Woburn, MA, USA) Each slide was then quantified by a specific quantification software.

The results showed cross-reactions between the species. For example, *epidermidis* amplicons hybridised on its capture probe give a value of 152, but give a value of 144, 9, 13 and 20 respectively for the S. *saprophyticus,* S. aureus, S. *haemolyticus* and S. hominis capture probes.

### Example 2: Detection of homologous FemA sequences on array bearing small specific capture nucleotide sequences

Protocols for capture nucleotide sequences immobilisation and silver staining detection were described in example 1 but the capture nucleotide sequences specific of the 5 *Staphylococcus* species were small oligonucleotides of 27 bases spotted at concentrations of 600 nM and are the following :

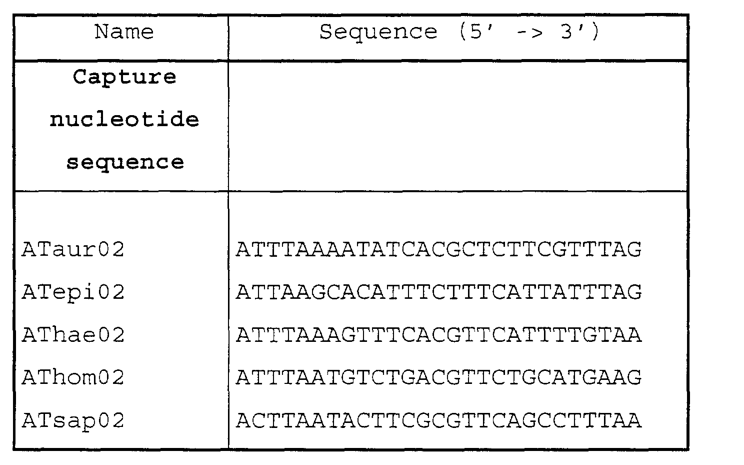

In this case, the targets are fragments of the FemA gene sequence corresponding to the different Staphylococci species which were amplified by a PCR using the following consensus primers :

APstap03: 5′ CCCACTCGCTTATATAGAATTTGA 3′

APstap04: 5′ CCACTAGCGTACATCAATTTTGA 3′
APstap05: 5′ GGTTTAATAAAGTCACCAACATATT 3′

This PCR was performed in a final volume of 100 *µ*l containing: 3 mM MgCl₂, 1 mM Tris pH 8, 1 µM of each primer, 200 µM of dACTP, dCTP and dGTP, 150 µM of dTTP, 50 *µ*M of biotin-16-dUTP, 2,5 U of Taq DNA polymerase (Boehringer Mannheim, Allemagne), 1 U of Uracil-DNA-glycosylase heat labile (Boehringer Mannheim, Allemagne), 1 ng of plasmid containing *FemA* gene. Samples were first denatured at 94°C for 5 min. Then 40 cycles of amplification were performed consisting of 1 min at 94°C, 1 min at 50°C and 1 min at 72°C and a final extension step of 10 min at 72°C. Water controls were used as negative controls of the amplification. The sizes of the amplicons obtained using these primers were 489 bp for all species.

The hybridisation solution was prepared as in example 1 and loaded on the slides. Slides were denatured at 98°C for 5 min. Hybridisation are carried out at 50°C for 2h. Samples are then washed 4 times with a washing buffer. The values were very low or almost undetectable and being always lower than 10% of the hybridisation controls performed on long capture probe.

### Example 3: Effect of the spacer length on the Sensitivity of detection of homologous FemA sequences on array bearing long capture nucleotide sequences with a small specific sequence

The experiment was conducted as described in example 2 with the same amplicons but the capture nucleotide sequences used were spotted from solutions at 600 nM and are the following:

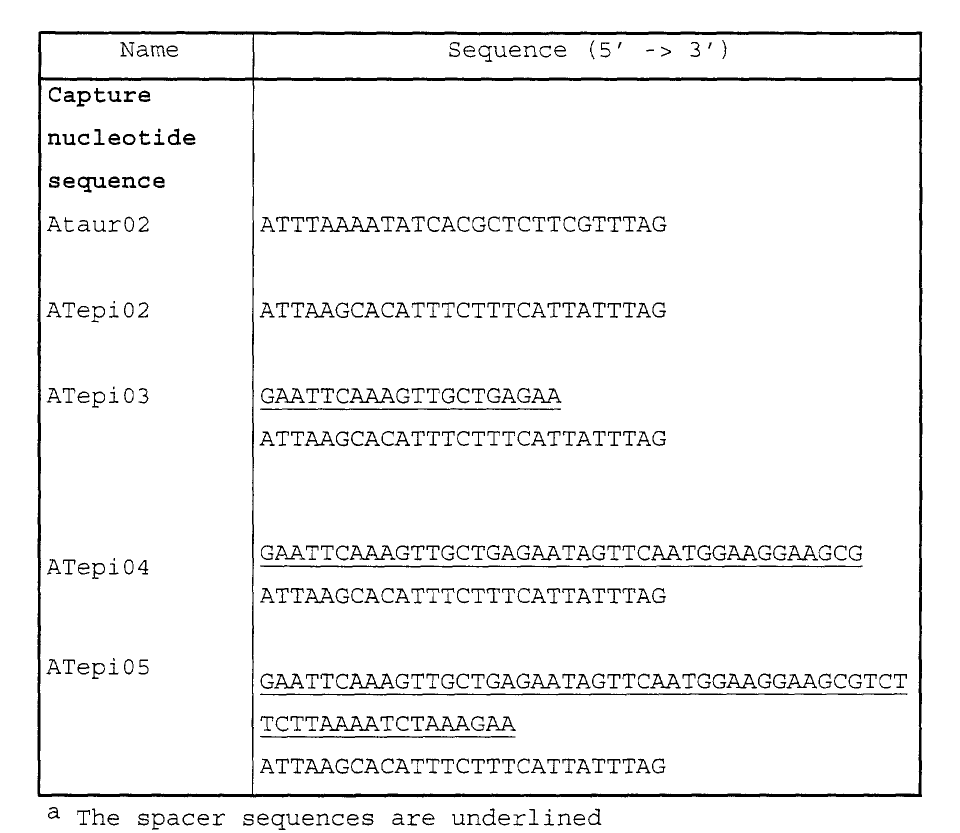

The target amplicons were 489 bp long while the capture nucleotide sequences were 27, 47, 67 or 87 bases single stranded DNA with a specific sequence of 27 bases.

The detection were made in fluorescence and colorimetry but only the last one is shown in figure 3. The values for the fixation controls were 217 and 161 for the hybridisation controls.

### Example 4: Effect of the capture nucleotide sequence concentration, on the sensitivity of detection of homologous FemA sequences on array bearing long capture nucleotide sequences with a small specific sequences

The experiment was conducted as described in example 3 with the capture nucleotide sequence ATepi03 having a spacer of 20 bases and spotted at concentrations ranging from 600 nM to 1800 nM. One of the data is given in Fig. 3.

### Example 5: Specificity of the detection of FemA sequences from different bacterial species on the same array bearing long capture nucleotide sequences with a small specific sequence (figure 4)

The experiment was conducted as described in example 2 but the capture nucleotide sequences were spotted at concentrations of 3000 nM and are the following:

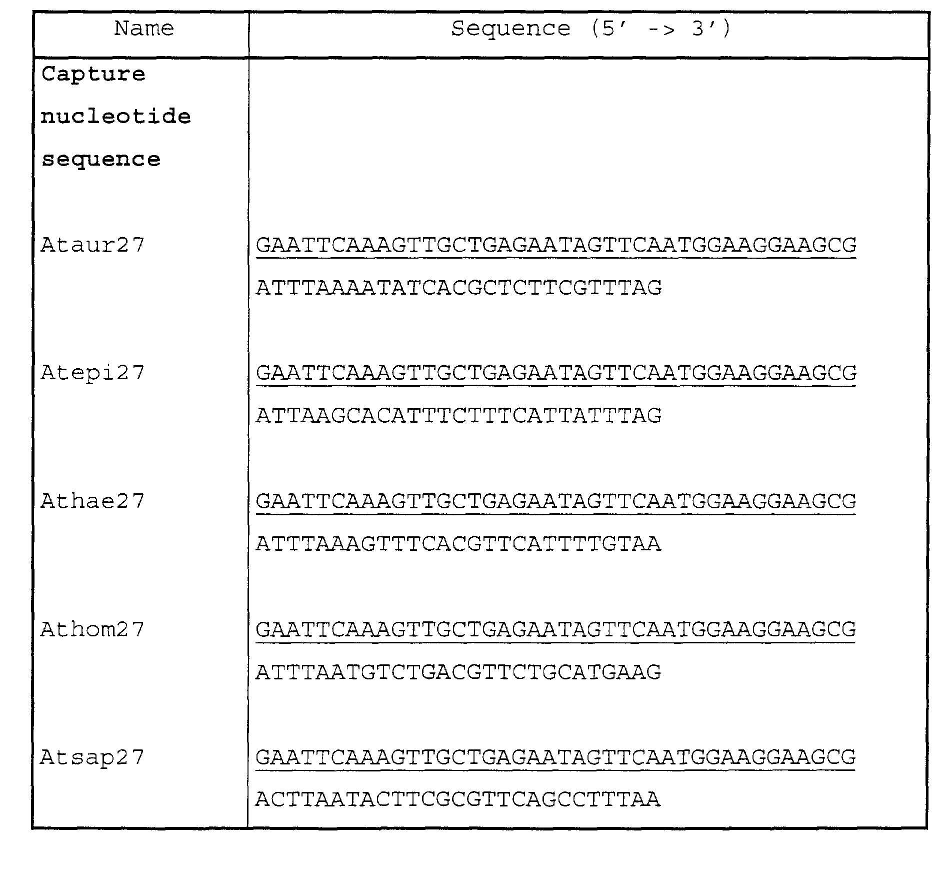

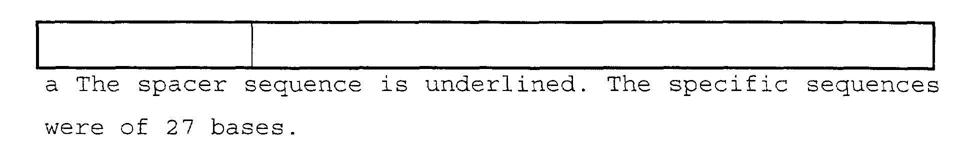

The targets are fragments of the FemA gene sequence corresponding to the different Staphylococci species which were amplified by PCR using the following consensus primers :

APcons3-1: 5' TAAYAAARTCACCAACATAYTC 3'
APcons3-2: 5' TYMGNTCATTTATGGAAGATAC 3'

A consensus sequence is present on the biochips which detects all the tested *Staphylococcus* species. All target sequences were amplified by PCR with the same pair of primers.

The size of the amplicons obtained using these primers were 587 bp for all species. The consensus sequence capture was a 489 base long single stranded DNA complementary to the target and identical to the sequence amplified in example 2. The detection was made in fluorescence. The PCR primers were able to amplify 15 various *Staphylococcus* species which were all detected on the consensus capture probe but not on the 5 specific capture probes described in this example which only fixed their corresponding sequence.

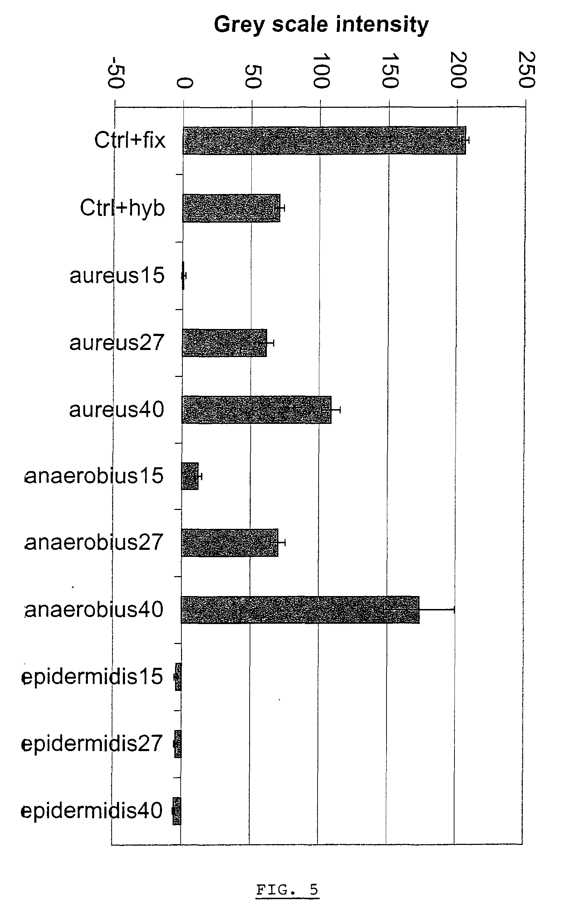

### Example 6: effect of the length of the specific sequence of the capture nucleotide sequence on the discrimination between homologous sequences (figure 5).

The experiment was conducted as described in example 5 but at a temperature of 43°C and the capture nucleotide sequences used are presented in the table here joined. The numbers after the names indicate the length of the specific sequences.

The *FemA* amplicons of *S*. *anaerobius* (a subspecies of *S*. *aureus)* were hybridised on an array bearing capture nucleotide sequences of 67 single stranded bases with either 15, 27 and 40 bases specific for the *S*.*aureus, anaerobius* and *epidermidis* at their extremities. The difference between the capture nucleotide sequences of *anaerobius* and *aureus* was only one base in the 15 base capture nucleotide sequence and 2 in the 27 and the 40 bases.

The amplicons of the *FemA* from the three *Staphylococcus* species were hybridised on the arrays. Only the results obtained with the *S. anaerobius* amplicons are shown in figure 5. Note that *anaerobius* specific capture probe of 15 bases could discriminate between the *anaerobius* and the aureus sequences which differ in this 15 bases sequence by one base.

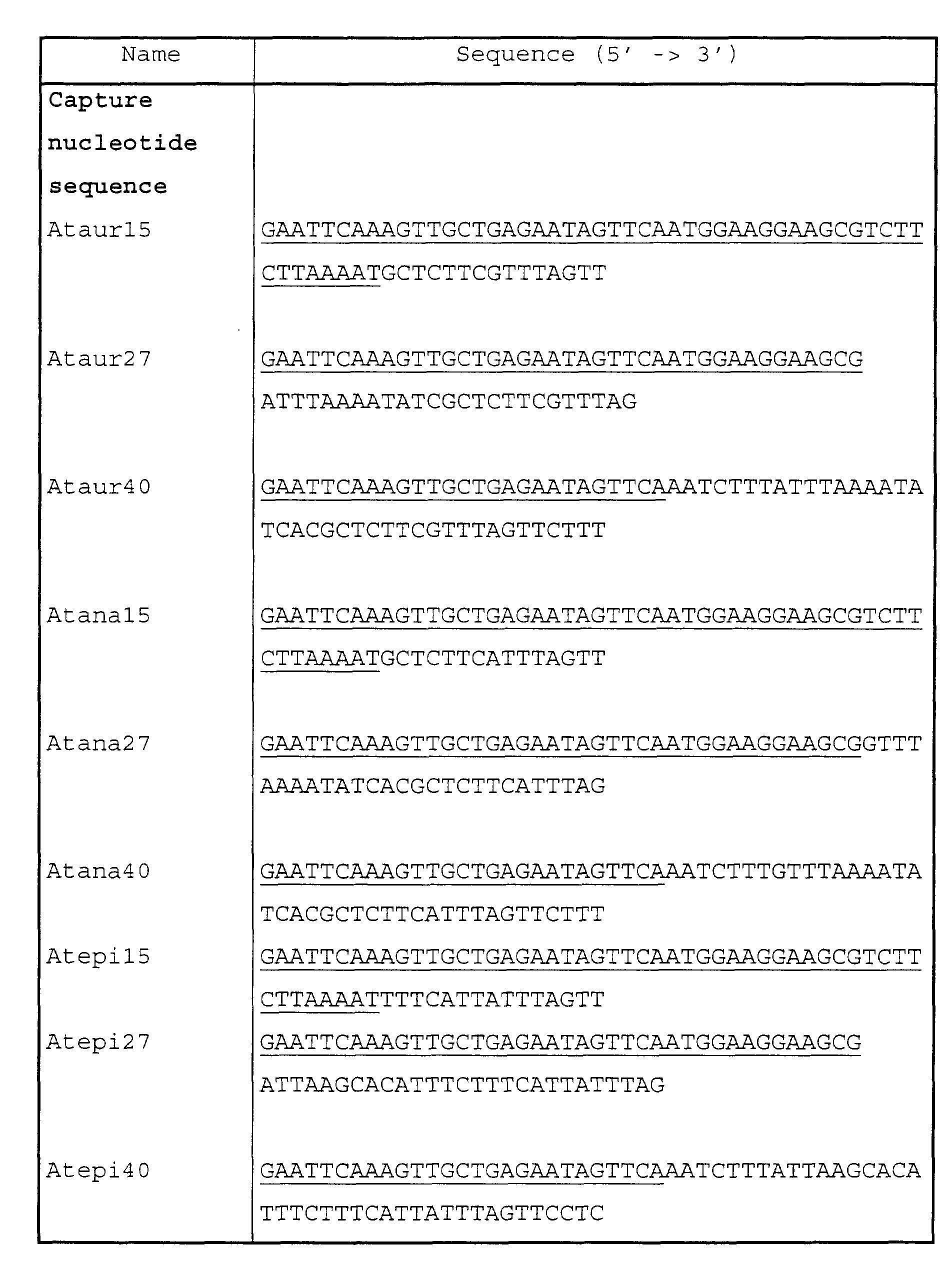

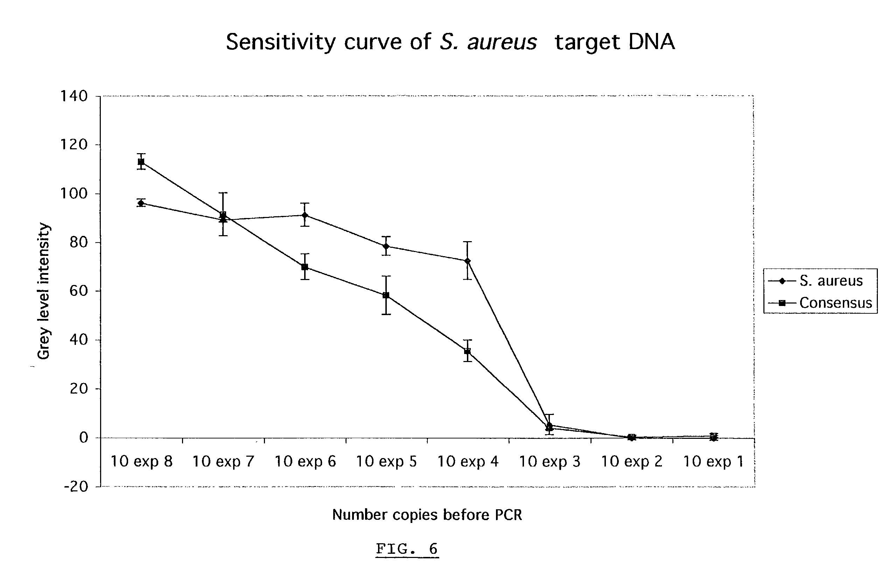

### Example 7: Sensitivity of the detection of FemA sequences of Staphylococcus aureus on arrays bearing specific sequence as proposed by this invention and the consensus sequence (figure 6)

The experiment was conducted as described in example 5 with the capture nucleotide sequences spotted at concentrations of 3000 nM. The bacterial FemA sequences were serially diluted before the PCR and being incubated with the arrays.

## Claims

1. Detection and/or quantification method of target nucleotide sequences, homologous to each other(s), and being possibly present in a biological sample, comprising the steps of:
- possibly extracting original nucleotide sequences (1) from the biological sample;
- possibly copying, amplifying and/or cutting at least a part of original nucleotide sequences present in the biological sample into target nucleotide sequences (2);
- putting into contact the possibly previously labelled target nucleotide sequences (2) with single stranded capture nucleotide sequences (3) bound to an insoluble solid support (4),
- detecting and/or quantifying a signal resulting from the hybridisation between the target and the capture nucleotide sequences (2,3) by complementary base pairing,
**characterised in that** said single stranded capture nucleotide sequences (3) contain a sequence (5) between about 10 and about 60 bases being specific for the target nucleotide sequences (2) and having a total length comprised between about 30 and about 600 bases,
and in that said single stranded capture nucleotide sequences (3) are bound to the insoluble support (4) according to an array with a density of at least 4 different single stranded capture nucleotide sequences/cm² of solid support (4) surface.

2. Detection and/or quantification method, **characterised in that** the copy or the amplification of homologous original nucleotide sequences (1) possibly present in the biological sample is first made with the same primer sequence(s) (6) prior to the detection.

3. Method according to claim 1 or 2, **characterised in that** the length of the capture nucleotide sequence (3) is comprised between about 40 and about 300 bases, preferably between about 40 and about 200 bases.

4. Method according to any of the claim 1 to 3, **characterised in that** the specific sequence (5) is separate from the surface of the solid support (4) by a spacer (7) having at least 6.8 nm.

5. Method according to any one of the preceding claims, **characterised in that** the capture nucleotide sequence (3) contains a sequence between about 15 and about 40 bases complementary to the target nucleotide sequence (2).

6. Method according to any one of the preceding claims, **characterised in that** the density of the capture nucleotide sequences (3) bound to the surface of the solid support (4) is superior to 3 fmoles per cm2 of solid support surface.

7. Method according to any one of the preceding claims, **characterised in that** the different target nucleotide sequences (2) to be detected present an homology between each other higher than 30%, preferably higher than 60%, more preferably higher than 80%.

8. Method according to any one of the preceding claims, **characterised in that** the capture nucleotide sequences (3) contain several portions (5) of bases specific for the target, each of these portions (5) containing between about 15 and about 40 bases complementary to the target nucleotide sequence (2), these portions (5) being separated from each other by at least 5 bases.

9. Method according to any one of the preceding claims, **characterised in that** the insoluble solid support (4) is selected from the group consisting of glasses, electronic devices, silicon supports, plastic supports, compact discs, filters, gel layers, metallic supports or a mixture thereof.

10. Method according to any one of the preceding claims, **characterised in that** the original nucleotide sequences (1) to be detected and/or be quantified are RNA sequences by the retro-transcription of the 3' or 5' end by using consensus primer and possibly a stopper sequence.

11. Method according to anyone of the preceding claims, **characterised in that** copied or amplified sequences are detected without previous cutting of original sequences into smaller portions.

12. Method according to any one of the preceding claims, **characterised in that** the original nucleotide sequences (1) to be detected and/or quantified are *FemA* genetic sequences of Staphylococci species selected from the group consisting of S. *aureus,* S. *epidermidis, S. saprophyticus, S. hominis* and/or S. *haemolyticus*.

13. Diagnostic and/or quantification kit, **characterised in that** it comprises means and media for performing the method according to any one of the preceding claims, preferably an insoluble solid support (4) upon which single stranded capture nucleotide sequences (3) are bound, said single stranded capture nucleotide sequences containing a sequence (5) of between about 10 and about 60 bases specific for a target nucleotide sequence (2) to be detected and/or quantified, said capture nucleotide sequence (3) having a total length comprised between about 30 and about 600 bases and being disposed upon the surface of the solid support (4) according to an array with a density of at least 4 single stranded capture nucleotide sequences/cm² of the solid support surface.

14. Diagnostic kit according to claim 13, **characterised in that** the insoluble solid support (4) is selected from the group consisting of glasses, electronic devices, silicon supports, plastic supports, compact discs, filters, gel layers, metallic supports or a mixture thereof.

15. Diagnostic kit according to claim 13 or 14, being biochips, for detection and/or quantification of at least 5 homologous DNA or RNA sequences (2) obtained after copying and/or amplification with one consensus primer(s) (6) and detection upon the solid support (4).

16. Diagnostic kit according to any one of the claims 13 to 15, being a biochips, for detection and/or quantification of 5 Staplylococcus species obtained after copying and/or amplification of one of their DNA or RNA sequences with one consensus primer(s) and detection on the solid support (4).

17. Diagnostic kit according to any one of the claims 13 to 16, being a biochips, for identification and/or quantification of 5 bacteria species obtained after copying and/or amplification of one of their DNA or RNA sequences with one consensus primer(s) and detection on the solid support (4).

18. Diagnostic kit according to any one of the claims 13 to 17, being a biochips, for identification and/or quantification of bacteria species together with the identification of the bacterial genus obtained after copying and/or amplification of one of their DNA or RNA sequences with one consensus primer(s) and detection on the solid support (4).
